# EUROPEAN PATENT SPECIFICATION

(11) **EP 3 123 970 B1**
(45) Date of publication and mention of the grant of the patent: **18.04.2018**
(21) Application number: 15460038.1
(22) Date of filing: 31.07.2015
(51) Int. Cl.: A61B 17/68, A61B 17/86, A61B 17/88

(54) **STABILIZATION OF TWO ADJACENT BONES**
STABILISIERUNG VON ZWEI BENACHBARTEN KNOCHEN
STABILISATION DE DEUX OS ADJACENTS

(43) Date of publication of application: 01.02.2017
(73) Proprietor: Ciupik, Lechoslaw Franciszek, 65-364 Zielona Gora (PL)
(72) Inventor: Ciupik, Lechoslaw Franciszek, 65-364 Zielona Gora (PL)
(74) Representative: PATERIS Patentanwälte PartmbB

(56) References cited:
- EP-A1- 2 626 019
- WO-A2-2004/069031
- DE-A1- 10 101 267
- US-A1- 2014 031 934

## Description

The present invention refers to an implant for immobilizing a joint between adjacent bones and, more particularly, to a sacroiliac implant. Furthermore the present invention refers to a tool for placing the implant and to a system comprising the implant and the tool.

The sacroiliac joint is subject to arthritic and aging changes like other arthrodial joints. Disorders of the sacroiliac joint can cause low back pain and radiating buttock and leg pain. Surgical treatment of these disorders may include immobilization of a joint. A number of prior art devices exist which may be used to stabilize or to fuse bones. This number comprises devices which are installed within the sacrum and the ilium.

The document EP1029519 discloses an implant which facilitates enhanced structural stability across a joint to be immobilized. The sacroiliac implant is an elongated structure having two cylindrical sections of differing diameters. The implant further has like-handed, but dissimilar pitched threaded sections at the proximal and distal end thereof to produce compressive forces across the joint. A hollow core traverses the length of the implant. A plurality of apertures or openings is formed through the implant transversely to the longitudinal axis, which communicate with the hollow core. The openings or apertures provide a conductive path of healing for bony ingrowth. Means are provided on the proximal end of the implant for engagement with a tool to drive the implant.

The document US 2009/0099610 discloses methods of stabilizing the sacroiliac joint by placing an expandable device in the joint to generate laterally opposing forces against the iliac and sacral surfaces of the sacroliliac joint in securely seat the device in a plane generally parallel to the sacroiliac joint. The expandable device is coated with or otherwise contains a bone material to promote fusion of the joint. The expandable device can be, for example, an expandable cage, a balloon-expandable stent or a self-expanding stent.

The document US 2011/0034957 discloses a bone anchoring device, which includes a bone anchoring element having a shank and a head, a continuous on-piece first locking element and a second locking element. The receiver member has a first chamber for receiving the dead, the dead being pivotable in the first chamber, and a second chamber with a channel for receiving the rod. A first locking element fixes the head in the first chamber at an angel, and a second locking element fixes the rod in the second chamber. The receiver member includes a body including both the first chamber and the second chamber. The head and the rod can be inserted a locked independently from each other. The head is fixed by exerting pressure onto it. The bone anchoring device is particularly applicable to the stabilization of the spine in the sacro-iliac and the cervical region.

The document US 2011/184519 discloses a sacro-iliac implant delivery tool including a first arm connected to a first cannula. The first cannula is rotatable relative to the first arm and is configured for support of a fastening element. A second arm is connected to the first arm. The second arm is rotatable relative to the first arm and is configured for detachable connection to a sacro-iliac implant. The second arm and the first cannula are configured for rotation relative to the first arm to a preset orientation such that the second arm is disposed along a first predetermined trajectory for delivering the sacro-iliac implant and the first cannula is disposed along a second predetermined trajectory for delivering the fastening element in alignment with the sacro-iliac implant for transarticular fixation.

The document US 2011/0184520 discloses a sacro-iliac implant system including at least one implant including a body defining an outer surface and being expandable in at least one dimension. The body is configured to expand in the at least one dimension from a first orientation to a second orientation such that the outer surface engages and spaces apart opposing articular surfaces of a sacro-iliac joint to fix the body with the articular surfaces.

The document US 2012/191191 discloses a sacro-iliac implant including a body extending from a first portion having an outer surface configured for fixation with a sacrum to a second portion having an outer surface being spaced apart and non-continuous with the outer surface of the first portion. A sleeve is disposed about the body and configured for implantation within at least an ilium. The sleeve extends from a first portion to a second portion having an inner surface and a flange disposed to engage an outer non-articular surface of the ilium. The inner surface of the second portion of the sleeve is engageable with the outer surface of the second portion of the body to cause axial translation of the body relative to the sleeve such that naturally separated articular surfaces of the sacrum and ilium are drawn into fixation to immobilize the sacroiliac joint.

The document US 2013/172736 discloses methods and apparatus for diagnostic and/or therapeutic manipulation of the sacroiliac joint. The sacroiliac joint may be immobilized by forming the fusion mass forms using at least one fixation member. In one embodiment, the fixation member comprises an external member and two internal members which threadedly engage each other. The fixation member is configured to expand and comprises bone growth promoting feature. The expanded member fixates the ilium to the sacrum on the side of the target sacroiliac joint, thus the sacroiliac joint is immobilized and fused.

The document US 2013/245703 discloses a fixation device for sacroiliac joint stabilization. The fixation device includes an elongated body comprising a bone anchor at a distal end. An axially moveable proximal anchor is carried by the proximal end of the fixation device. In one embodiment, the device is inserted through the ilium of the pelvis and the bone anchor is rotated into position within the sacrum. The proximal anchor is distally advanced with respect to the bone anchor to provide compression across the sacroiliac joint. The document WO 2014/018240 discloses a sacro-iliac implant including an inner member having an inner surface and an outer surface. The inner member extends between a first end and a second end configured for penetrating a sacrum. An outer member extends between a first end including a flange and being configured to engage an outer non-articular surface of an ilium and a second end. The inner member is rotatable relative to the outer member such that the outer surface of the inner member adjacent its first end engages the outer member to cause axial translation of the inner member relative to the outer member in a configuration such that separated articular surfaces of the sacrum and the ilium are drawn into fixation.

The document WO 2004/069031 disclosed a compression screw apparatus. The compression screw includes a primary screw and a secondary screw. The primary screw includes an at least partially threaded leading portion and an at least partially threaded trailing portion. The secondary screw included outer threads and has a central opening which is also threaded and adapted for being screwed onto and matingly engaging the trailing portion of the primary screw. The first element comprises at least one elevated circumferential structure which is segmented into at least one shelf in a position where a channel-like structure is crossing the circumferential structure.

It is a general objective of the present invention to provide an advantageous implant for stabilization and fusion of two adjacent bones, particularly of a sacrum and an ilium. The objective is solved by an implant with features according to claim 1. Further advantageous embodiments of the invention are contained in the further independent and dependent claims, the figures and the examples.

A first aspect of the invention is related to an implant for immobilizing a joint between a first bone and a second bone, comprising at least a first element and a second element, wherein the first element has a cylindrical body comprising:
a proximal section having a first thread,
a distal section comprising at least one elevated circumferential structure, and
an intermediate section interconnecting said proximal and distal section,
wherein the proximal, distal and intermediate section have an external surface,
wherein the second element has a cylindrical body comprising:
an external surface having a second thread,
an internal surface having a third thread with the same pitch as the first thread, and wherein the diameter of the internal surface of the second element is the same as the diameter of the external surface of the proximal section of the first element,
wherein the circumferential structure is segmented into at least one shelf in a position where the channel-like structure is crossing the circumferential structure, and wherein the shelf comprises at least one cutting projection,
characterized in that
the intermediate section of the first element has at least one recess in the external surface forming a reservoir suitable for bone material which is separated from a bone upon an implantation process,
wherein at least one channel-like structure is running from the front end of the first element to the reservoir.

The inventive implant is advantageous because it facilitates the stabilization and fusion of two adjacent bones, particularly of a sacroiliac joint. A further advantage is that the design of the shelfs allows both an introduction of the first element into the bone material and an anchoring of the first element in the bone material. The inventive implant is further advantageous because bone material, separated from the bone upon an implantation process, can be transported away from the front end of the first element through the channel-like structure. A further advantage of the inventive implant is that the bone material can be collected in the reservoir formed by the recess in the external surface of the intermediate section. A further advantage of the inventive implant is that it can be placed by use of one single tool.

The design of the second element is advantageous because the second thread facilitates a setting of the implant by screwing of the second element into the second bone, after setting of the first element into the first bone which is facilitated by the second thread. Moreover, the second element can be moved relatively to the first element by means of the third thread functioning as an internal thread mating with the complementary first thread.

The recess forming the reservoir is a hollow or a depression in the external surface of the intermediate section of the implant.

The term "elevated" in the context of the circumferential structure means that it is extended over the external surface of the distal section of the first element in the radial direction.

The term "cutting projection" is referring to means which are formed for facilitating a cutting process. This term comprises cutting blades but has a more general meaning.

The channel-like structure preferably has a helical form which is crossing the ridges of the second thread. This form advantageously supports the transport of bone material from the front end. It can, however, also have a straight form which is running parallel to the longitudinal axis of the implant.

In accordance with the construction the front end of the inventive implant is formed by the front end of the first element. It is preferred if the front end is formed as a cutting head comprising at least one cutting blade with at least one cutting edge. The cutting blades facilitate advantageously the movement of the implant, particularly of the first element, into the bone material by application of force, thereby separating material from the bone. It is further preferred that the cutting blade of the front end is arranged relatively to the channel-like structure in a way that bone material removed by the cutting blade will be pushed directly into the channel-like structure.

It is preferred if the second element comprises at least one recess arranged at its end facing the intermediate section, wherein the recess comprises cutting projections. The second element can comprise further advantageous cutting projections at the end facing the intermediate section for cutting bone material and drilling the second element into the bone. Upon an implanting procedure, bone material removed by the second element will be pushed through by the second element towards the reservoir and subsequently into the space between two adjacent bones the implant is introduced in.

In a preferred embodiment of the invention, the reservoir in the intermediate section comprises means which are formed for directing a flow of material from the reservoir into a space between the first and the second bone. These means are structures which are directing the movement of the bone material from the reservoir into the space between the bones upon an implantation procedure. The means are advantageous because the distribution of bone material into the space between the first and second bone supports the immobilization of the joint between the first and second bone.

It is further preferred if at least a part of the proximal, distal and / or intermediate sections comprises a cavity formed by an internal surface, wherein apertures are connecting the internal and external surface of the first element. The hollow structure of at least a part of the first element makes the implant lighter by weight than a comparable massive first element. It is further preferred that a mesh built by struts is formed in the cavity of the first element. The struts advantageously contribute to the stability of the otherwise hollow structure. The apertures and the mesh advantageously allow bony ingrowth through the apertures to the cavity. The cavity can be filled with one or more substances which encourage bony ingrowth. The second element can also comprise apertures. If the first and / or second element comprises apertures, the apertures are ideally not present in the shelf or in the threads, which have preferably a solid structure without apertures.

The inventive implant further preferably comprises means for accommodating a tool which is formed for driving the implant. It is particularly preferred if both the first and the second element of the implant comprise means for accommodating an tool which is formed for driving the first or second element alone or both the first and second element together. Ideally the means for accommodating the tool and the tool have complementary forms, so that the tool is positively insertable into the means.

A second aspect of the present invention is related to a tool having means for connecting to an implant according to the present invention, comprising:
a tang,
a sleeve,
a locking knob,
a sleeve knob,
a latch, and
a handle.

The tool has the appearance of a screwdriver. The tang has preferably means for connecting it to the proximal section of the first element of the implant. It is further preferred if the sleeve has means for connecting it to the second element of the implant. The means of the tang and the sleeve are ideally complementary to the means of the first and second element, respectively, so that they can be positively connected.

The locking knob is provided for setting the tool into a defined working position. A first working position of the tool can preferably be set by engaging the locking knob, for instance by pushing it in. In this position both the sleeve and the tang can be moved together by turning the handle. This advantageously facilitates a combined rotation of the first and second element of the inventive implant. A second working position can preferably be set by releasing the locking knob. In the second working position the second element can be moved independently of the first, and only the second element will be moved by turning the handle. The sleeve knob is provided for disconnecting the tool from the implant when necessary.

A third aspect of the present invention is related to a system of an implant and a tool according to the invention. Also described herein is a method for treating a joint comprising a first bone and a second bone, the method comprising the steps of:
providing an implant with a first element and a second element according to the present invention, wherein the first and second elements are connected to each other,
determining a trajectory for introduction of the implant,
connecting the implant with a tool according to the present invention, wherein the first element of the implant is connected to a tang of the tool and the second element of the implant is connected to a sleeve of the tool,
setting the tool into a first working position with a move of the locking knob,
delivering the implant to a prepared site for introducing the implant so that the pointed head of the first element is contacting the first bone, introducing the implant into the first bone and the second bone by applying force on the handle until the reservoir of the first element is contacting the second bone,
disengaging the locking knob using the latch, thereby setting the tool into a second working position,
screwing the second element into the first bone by applying force to the handle of the tool until the second element is completely within the first bone,
disconnecting the tool from the implant by engaging the sleeve knob. The introduction of the first element into the bones can be carried out by a screwing motion as well as by pushing it through the bones.

In a preferred variation of the method the joint is a sacro-iliac joint, wherein the first bone is the ilium and the second bone is the sacrum. Embodiments of the invention are described in conjunction with the accompanying figures.
- Figure 1: shows an implant according to the present invention.
- Figure 2: shows a head of a first element of the implant according to Fig. 1.

- Figure 3a: shows a longitudinal cut of the intermediate section of the implant according to Fig. 1
- Figure 3b: shows a cross section of the intermediate section of the implant according to Fig. 1
- Figure 4: shows the implant according to Fig. 1 as placed into two bones
- Figure 5: shows a tool for placing the implant according to Fig. 1.
- Figure 6: shows the tip of the tool according to Fig. 5.
- Figure 7: shows a flow chart of a method of using a system according to the present invention.

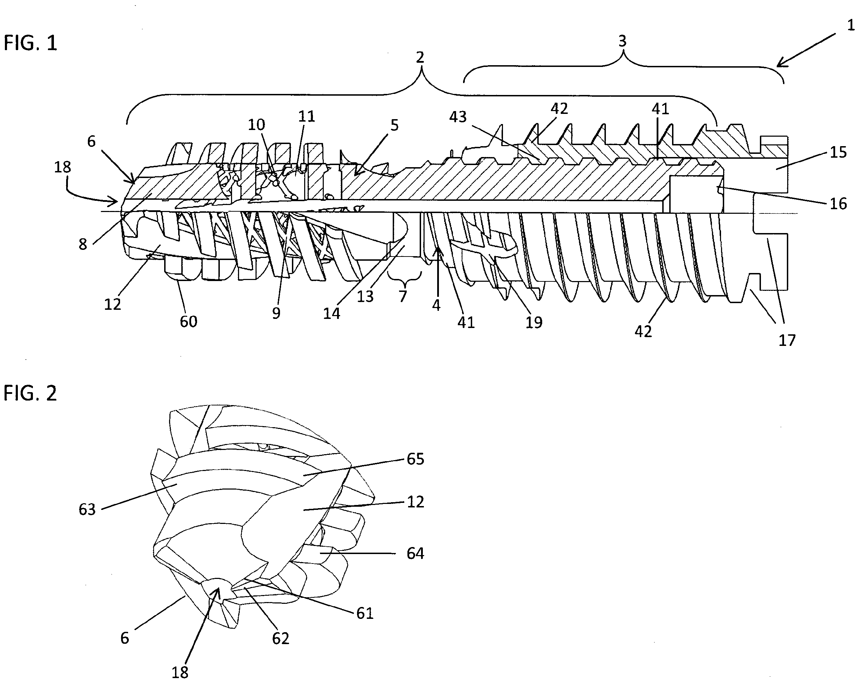

The sacroiliac implant 1 of this invention is shown in Fig. 1 as an elongate implant consisting of a first element 2 and a second element 3. The first element 1 has proximal section 4 and a distal section 5. The front end 6 of the distal section 5 is formed as a cutting head. An intermediate section 7 is located between the proximal 4 and the distal section 5. The first element 2 comprises an external surface 8 and an internal surface 9. The internal surface 9 is forming a contiguous surface defining a hollow structure, or cavity, within the proximal 4, distal 5, and intermediate section 7. The hollow structure of the first element 2 is stabilized by a mesh formed by a number of struts 10. A plurality of openings or apertures 11 is connecting the external 8 and internal surface 9. The openings or apertures 11 and the mesh of struts 10 provide conductive pathways by which bony ingrowth into the implant can be achieved.

In an embodiment of the inventive implant, the first element 2 may have a cavity with an internal surface in the distal section 5, but not in the intermediate section 7 and / or in the proximal section 4, so that apertures 11 would only be provided for the distal section 5. It is also possible that the intermediate section 7 and / or the proximal section 4 have a cavity and the distal section 5 is massive. Alternatively, the first element 2 may have a massive structure at all, so that no internal surface would be provided, and consequently no apertures.

The front end 6 is shown in more detail in Fig. 2. The cutting head of the front end 6 is supporting its function of drilling and screwing the way of the implant into a bone upon an implantation process. A number of three cutting blades 61 are arranged at the front end 6. Alternatively, any suitable number of cutting blades 61 can be arranged which support the drilling of the first element 2 and thus of the whole implant 1 into the bone. Each cutting blade 61 has a cutting edge 62. A channel-like structure 12 starts immediately at the front end 6 which serves for transport of bone material away from the drilling site after it has been separated from the bone by the cutting blade 61. The starting point of the channel-like structure 12 is arranged relatively to the cutting blade 61 in such a way that material separated from the bone is received immediately by the channel-like structure 12. The channel-like structure 12 is formed with a roundish and even surface. The depth and width of the channel-like structure 12 are suited for a flow of bone material. The depth and width of the channel-like structure 12 are preferably the same over its length. Alternatively the dimensions of the channel-like structure 12 can also vary over its length, e.g. the channel-like structure 12 can be wider at the front end 6 and narrower in the proximity of the intermediate section 7. The path of the channel-like structure 12 is preferably formed helically, but could alternatively also run parallel to the longitudinal axis of the first element 2. The angle between the channel-like structure 12 and the longitudinal axis of the implant is preferably between 0 and 50°, and more preferred between 10 and 45°, and more preferred between 20 and 40°. There are preferably three channel-like structures 12 arranged at the first element 2, however it is possible to arrange any suitable number of channel-like structures 12.

In Fig. 1 a number of elevated circumferential structures 60 is shown which is running around the distal section 5 of the first element 2. The elevated circumferential structure 60 can be formed as a ring which is arranged in an angle of 90°C to the longitudinal axis of the implant 1, so that the upper and lower surface of the ring structure are parallel to each other and in an angle of 90°C to the longitudinal axis of the implant 1. Alternatively the circumferential structure 60 can have a slope between 0 and 90° to the longitudinal axis of the implant. As shown in Fig. 1, more than one elevated circumferential structure is arranged at the distal section 5. The first element 2 can comprise any suitable number of elevated circumferential structures 60. It is preferred if the first element 2 has between 1 to 15 elevated circumferential structures, more preferred 3 to 12, and yet more preferred 5 to 10. The elevated circumferential structures 60 can be evenly spaced from each other. Alternatively, the elevated circumferential structures 60 can be differently spaced from each other, so that at least two elevated circumferential structures 60 have a different space between each other that the other elevated circumferential structures 60. In a further alternative embodiment the elevated circumferential structure can be a helix, so that it has screw-like appearance.

By crossing through the elevated circumferential structure 60 the channel-like structure 12 is forming shelfs 63 from the elevated circumferential structure 60 (Fig. 2). The brinks of the shelfs 63 at the crossing points of the channel-like structure 12 are formed as cutting projections 64 at one side and as locking projections 65 at the other side. The shelfs 63 are anchoring elements which also have a cutting function. The shelfs 63 can have various shapes, e.g. as seen in a cross section they can have a strong rectangular form or comprise roundish parts. The shelfs can have a slope which results from the original slope of the elevated circumferential structure 60.

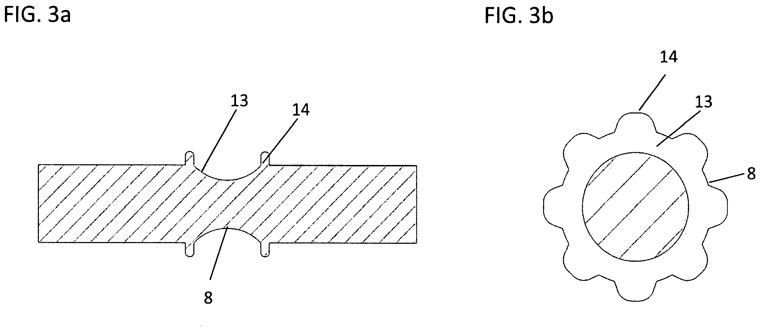

In Fig. 1 a reservoir 13 is shown which is formed in the external surface of the intermediate section 7. The reservoir 13 is a recess provided for receiving bone material which has been separated from the bone. The bone material is transported away from the front head 6 through the channel-like structure 12 which is running from the front end 6 to the reservoir 13. The reservoir 13 has the form of a hollow; it can be formed as a continuous hollow around the intermediate section or, alternatively, be divided into several smaller "sub"-reservoirs which appear as separate hollows in the external surface of the intermediate section 7. Means 14 are provided at the brinks of the reservoir 13 which support the dispersal of bone material from the reservoir 13 into the space between two bones the implant 1 is placed in. The means 14 are formed as projections which give the flow of bone material from the reservoir 13 a certain direction. The means 14 as shown in Fig. 3a and Fig. 3b have a roundish form with similarity to semicircles. Alternatively the means 14 could have a spiky form, or any other form suitable for supporting the dispersal of bone material. If the reservoir 13 is a continuous hollow, the means 14 are provided at both sides of the reservoir 13 and along its brinks around the intermediate section 7. If several small reservoirs are provided, means 14 can be provided around each reservoir.

The proximal part 4 of the first element 2 has a first thread 41. Apertures are ideally not present in the threads. The second element 3 comprises means 17 for accommodating a tool 30. A tunnel 18 for application of a guide wire is running through both the first 2 and the second element 3 of the implant.

The second element 3 as shown in Fig. 1 has an external surface 15 and an internal surface 16. The second element 3 can also have apertures (not shown). The second element 3 also comprises means 17 for accommodating a tool 30. A recess 19 is formed at the end of the second element 3 facing the intermediate section 7 of the first element 2. The recess 19 comprises cutting projections, such as cutting blades. Further cutting projections can be arranged at the same end of the second element 3, but outside the recess 19 (not shown). Upon an implantation process, bone material separated by the second element 3 will be pushed towards the intermediate section 7 and received by the reservoir 13, and subsequently transferred into the space between the two adjacent bones.

The external surface 15 has a second thread 42, and the internal surface 16 has a third thread 43. The pitch of the third thread 43 is of the same size as the pitch of the first thread 41, and the diameter of the internal surface 16 of the second element 3 is the same as the diameter of the external surface 8 of the proximal section 4 of the first element 2. The third thread 43 functions as an internal thread mating with the complementary first thread 41, so that this way the second element can be moved relatively to the first element by way of a screwing motion of the second element 3 around the first element 2. If the second element 3 has openings or apertures, they are not present in the threads.

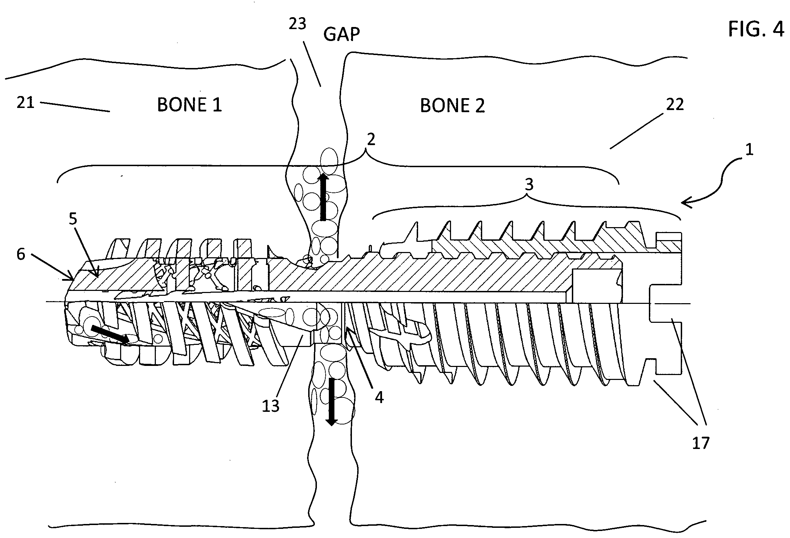

The position of the implant 1 within two adjacent bones is shown in Fig. 4. The first element 2 is placed into a first bone 21 and the second element 3 is placed into a second bone 22. If the implant 1 is a sacroilial implant, the first element 2 is placed into the sacrum and the second element 3 into the ilium. Bone material, removed from the first bone 21 upon the implantation process and stored in the reservoir 13, can be dispersed into the space 23 between the first bone 21 and the second bone 22. The thick arrows indicate the transport and movement, respectively, of the bone material.

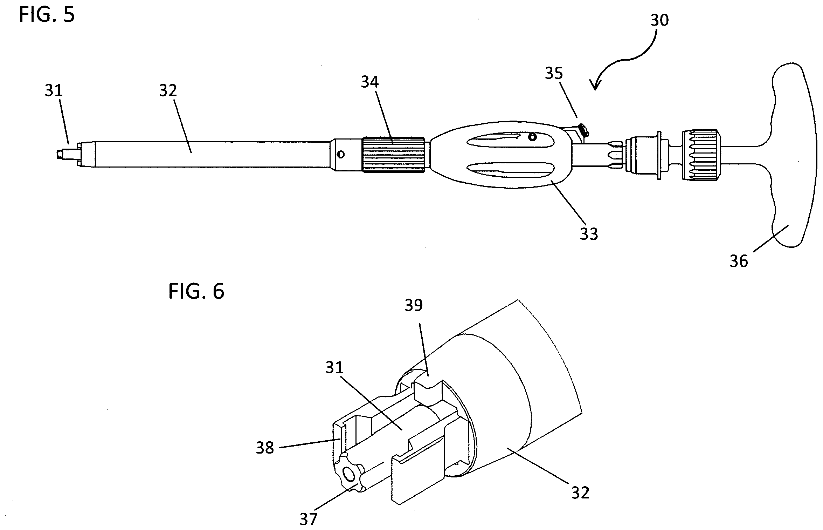

A tool 30 as shown in Fig. 5 is provided for placing the implant into two adjacent bones. The tool 30 has a tang 31, a sleeve 32, a locking knob 33, a sleeve knob 34, a latch 35, and a handle 36. The long form of the tool 30 facilitates an efficient connection of the tang 31 to the first element 2 as well as of the sleeve 32 to the second element 3 of the implant 1. The tang 31 has connecting elements 37 which are provided for transmitting the torque from the tool 30 to the first element 2. As shown in Fig. 6, the connecting element 37 is formed as a five-pointed star. Alternatively it could also have other forms, for example an oval form, a square form, or a hexagonal form. Complementary forms are provided in the first element 2 for a positive connection of the tool 30 to the first element 2 of the implant 1. Additionally the tang 31 comprises forms like a clamp 38. The clamp 38 functions as an anchor in order to catch the first element 2.

The sleeve 32 has connecting elements 39 which are provided for connecting the tool 30 to the second element 3. As shown in Fig. 6, the connection element 39 has a square form. Alternatively, it could also have other forms, like a triangular or an oval form. Complementary forms are provided in the second element 3 for a positive connection of the tool 30 to the second element 3 of the implant 1.

The tang 31 is pushed by a spring mechanism which is operated by the handle 36. The tang 31 and the sleeve 32 are set into a first working position by engaging the locking knob 33. In the first working position of the tool 30, the tang 31 and the sleeve 32 are "locked", which means that they cannot be moved relatively to each other, and are both turned by rotational movement of the handle 36. The locking knob 33 can be disengaged by pushing the latch 35, setting the tool 30 in a second working position. In the second working position, the second element 3 can be moved relatively to the first element 2. The sleeve knob 34 is provided for disconnecting the tool 30 from the implant 1. Other working positions are possible, like a third working position in which only the tang 31 will be actively moved.

The implant 1 and the tool 30 form a system which is used for placing the implant 1 into two bones in order to stabilize a joint between the two bones.

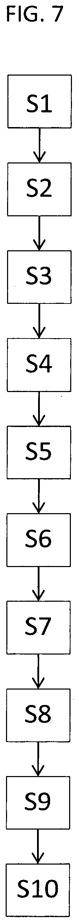

In a first step S1 of a method of placing an implant into a sacrum and an ilium, as shown in Fig. 7, an implant 1 is provided which has a first element 2 and a second element 3. The implant 1 is selected according to the size of the bones it shall be implanted in. The first 2 and second elements 3 are connected by means of the threads 41 and 43. In a second step S2 a trajectory of introducing the implant 1 into the bones is determined. It is possible to prepare a hole through the bones which is however smaller than the diameter of the first element 2. A Kirschner wire is inserted in the trajectory, which is also running through the tunnel 18 of the inventive implant 1. In a third step S3 a dilator is inserted. Steps S2 and S3 are carried out for preparation of the site and the bones for placing the implant 1. In a fourth step S4 the implant 1 is connected to a tool 30, wherein the first element 2 is connected to the tang 31of the tool 30 and the second element 3 is connected to a sleeve 32 of the tool 30. In a fifth step S5 the tool 30 is set into a first working position by engaging the locking knob 33.

In a sixth step S6 the implant is delivered to the site prepared for introducing the implant so that the front end 6 of the first element is contacting the ilium. In a seventh step S7 the implant 1 is introduced into the ilium and the sacrum by applying at least a lateral force on the handle 36, so that the implant 1 is pushed through the bones, until the reservoir 13 of the first element 2 is contacting the sacrum. In this position a torque is applied to the first element 2 by means of the tool 30, so that the implant 1 makes a rotational movement around its longitudinal axis so that the shelfs 63 of first element 2 are driven into the bone. This process is supported by the cutting projections 64 of the shelfs 63. The locking projections 65 support the anchoring of the first element 2 in the sacrum. Alternatively a torque could be applied in addition to the lateral force, so that a screwing motion of the first element 2 is carried out already during introducing of the first element 2 into the bones.

In an eighth step S8 the locking knob 33 is disengaged using the latch 35, thereby setting the tool into a second working position. In a ninth step S9 the second element is screwed into the ilium by applying both a torque and a lateral force on the handle 36 of the tool until the second element is completely within the second bone. In a tenth step S10 the tool 30 is disconnected from the implant 1 by engaging the sleeve knob 34.

### List of reference signs

1 = implant
2 = first element
3 = second element
4 = proximal section of the first element
5 = distal section of the first element
6 = front end
7 = intermediate section of first element
8 = external surface of first element
9 = internal surface of first element
10 = struts
11 = aperture
12 = channel-like structure
13 = reservoir
14 = means for directing flow of material
15 = external surface of second element
16 = internal surface of second element
17 = means for accommodating a tool
18 = tunnel for guide wire
19 = recess
21 = first bone
22 = second bone
23 = space between first and second bone
30 = tool
31 = tang
32 = sleeve
33 = locking knob
34 = sleeve knob
35 = latch
36 = handle
37 = connecting element of tang
38 = clamp
39 = connecting element of sleeve
41 = first thread
42 = second thread
43 = third thread
60 = elevated circumferential structure
61 = cutting blade
62 = cutting edge
63 = shelf
64 = cutting projection
65 = locking projection

## Claims

1. Implant (1) for immobilizing a joint between a first bone and a second bone, comprising at least a first element (2) and a second element (3), wherein the first element (2) has a cylindrical body comprising:
a proximal section (4) having a first thread (41),
a distal section (5) comprising at least one elevated circumferential structure (60), and
an intermediate section (7) interconnecting said proximal (4) and distal section (5), wherein the proximal (4), distal (5) and intermediate section (7) have an external surface (8),
wherein the second element (3) has a cylindrical body comprising:
an external surface (15) having a second thread (42),
an internal surface (16) having a third thread (43) with the same pitch as the first thread (41), and wherein the diameter of the internal surface (16) of the second element (3) is the same as the diameter of the external surface (8) of the proximal section of the first element (2),
wherein the intermediate section (7) of the first element (2) has at least one recess in the external surface (8) forming a reservoir (13) suitable for bone material which is separated from a bone upon an implantation process,
wherein at least one channel-like structure (12) is running from the front end (6) of the first element (2) to the reservoir (13), and
wherein the circumferential structure (60) is segmented into at least one shelf (63) in a position where the channel-like structure (12) is crossing the circumferential structure (60), and wherein the shelf (63) comprises at least one cutting projection.

2. Implant (1) according to claim 1, **characterized in that** the front end (6) is formed as a cutting head comprising at least one cutting blade (61) with at least one cutting edge (62).

3. Implant (1) according to claim 2, **characterized in that** the cutting blade (61) of the front end is arranged relatively to the channel-like structure (12) in a way that bone material removed by the cutting blade (61) will be pushed directly into the channel-like structure (12).

4. Implant (1) according to any of the foregoing claims, **characterized in that** the second element comprises at least one recess (19) arranged at its end facing the intermediate section, wherein the recess (19) comprises cutting projections.

5. Implant (1) according to any of the foregoing claims, **characterized in that** the reservoir (13) in the intermediate section (7) comprises means (14) which are formed for directing a flow of material from the reservoir (13) into a space between the first and the second bone upon an implantation process.

6. Implant (1) according to any of the foregoing claims, **characterized in that** at least a part of the proximal (4), distal (5) and / or intermediate sections (7) comprises a cavity formed by an internal surface (9), wherein apertures (11) are connecting the external (8) and internal surface (9) of the first element.

7. Implant (1) according to claim 6, **characterized in that** a mesh of struts (10) is formed in the cavity of the first element (2).

8. Implant (1) according to any of the foregoing claims, **characterized in that** the first element (2) and the second element (3) both have means (17) for accommodating a tool which is formed for driving the implant.

9. System comprising an implant (1) according to any of claims 1 - 8 and a tool (30) comprising
a tang (31) with means (37, 38) for connecting to the proximal section of the first element of the implant (2),
a sleeve (32) with means (39) for connecting to the second element of the implant (2),
a locking knob (33) for setting the tool (30) into a defined working position,
a sleeve knob (34) for disconnecting the tool (30) from the implant (1),
a latch (35) for disengaging the locking knob (33), and
a handle (36) for turning the tang (31) and/or the sleeve (32).

10. System according to claim 9, **characterized in that** a first working position of the tool (30) can be set by engaging the locking knob (33).

11. System according to claim 9 or 10, **characterized in that** a second working position of the tool (30) can be set by releasing the locking knob (33).

## Patentansprüche

1. Implantat (1) zum Immobilisieren eines Gelenks zwischen einem ersten Knochen und einem zweiten Knochen, umfassend mindestens ein erstes Element (2) und ein zweites Element (3), wobei das erste Element (2) einen zylindrischen Körper aufweist, umfassend:
einen proximalen Abschnitt (4) mit einem ersten Gewinde (41),
einen distalen Abschnitt (5), umfassend mindestens eine erhöhte umlaufende Struktur (60), und
einen Zwischenabschnitt (7), der den proximalen (4) und distalen (5) Abschnitt verbindet, wobei der proximale (4), distale (5) und Zwischenabschnitt (7) eine äußere Oberfläche (8) aufweisen,
wobei das zweite Element (3) einen zylindrischen Körper aufweist, umfassend:
eine äußere Oberfläche (15) mit einem zweiten Gewinde (42),
eine innere Oberfläche (16) mit einem dritten Gewinde (43) mit derselben Ganghöhe wie das erste Gewinde (41) und wobei der Durchmesser der inneren Oberfläche (16) des zweiten Elements (3) der gleiche ist wie der Durchmesser der äußeren Oberfläche (8) des proximalen Abschnitts des ersten Elements (2),
wobei der Zwischenabschnitt (7) des ersten Elements (2) mindestens eine Einbuchtung in der äußeren Oberfläche (8) aufweist, die ein Reservoir (13) bildet, das für Knochenmaterial geeignet ist, das während eines Implantationsvorgangs von einem Knochen abgetrennt wird,
wobei sich mindestens eine kanalähnliche Struktur (12) vom vorderen Ende (6) des ersten Elements (2) zu dem Reservoir (13) erstreckt, und
wobei die umlaufende Struktur (60) in einer Position, an der die kanalähnliche Struktur (12) die umlaufende Struktur (60) kreuzt, in mindestens eine Nase (63) aufgeteilt ist, und wobei die Nase (63) mindestens einen Schneidevorsprung umfasst.

2. Implantat (1) gemäß Anspruch 1, **dadurch gekennzeichnet, dass** das vordere Ende (6) als Schneidkopf ausgebildet ist, umfassend mindestens eine Schneideklinge (61) mit mindestens einer Schneidekante (62).

3. Implantat (1) gemäß Anspruch 2, **dadurch gekennzeichnet, dass** die Schneideklinge (61) des vorderen Endes relativ zur kanalähnlichen Struktur (12) in einer Weise angeordnet ist, dass Knochenmaterial, das von der Schneideklinge (61) entfernt wird, direkt in die kanalähnliche Struktur (12) geschoben wird.

4. Implantat (1) gemäß einem der vorangehenden Ansprüche, **dadurch gekennzeichnet, dass** das zweite Element mindestens eine Einbuchtung (19) umfasst, die an seinem Ende, welches in Richtung des Zwischenabschnitts weist, angeordnet ist, wobei die Einbuchtung (19) Schneidevorsprünge umfasst.

5. Implantat (1) gemäß einem der vorangehenden Ansprüche, **dadurch gekennzeichnet, dass** das Reservoir (13) in dem Zwischenabschnitt (7) Mittel (14) umfasst, die dazu ausgebildet sind, einen Materialfluss vom Reservoir (13) in einen Raum zwischen dem ersten und dem zweiten Knochen während eines Implantationsvorgangs zu leiten.

6. Implantat (1) gemäß einem der vorangehenden Ansprüche, **dadurch gekennzeichnet, dass** mindestens ein Teil der proximalen (4), distalen (5) und/oder Zwischenabschnitte (7) einen Hohlraum umfasst, der von einer inneren Oberfläche (9) gebildet wird, wobei Öffnungen (11) die äußere (8) und innere (9) Oberfläche des ersten Elements verbinden.

7. Implantat (1) gemäß Anspruch 6, **dadurch gekennzeichnet, dass** ein Netz aus Streben (10) in der Höhle des ersten Elements (2) gebildet ist.

8. Implantat (1) gemäß einem der vorangehenden Ansprüche, **dadurch gekennzeichnet, dass** sowohl das erste Element (2), als auch das zweite Element (3) Mittel (17) zum Aufnehmen eines Werkzeugs aufweisen, das zum Antreiben des Implantats ausgebildet ist.

9. System, umfassend ein Implantat (1) gemäß einem der Ansprüche 1 bis 8 und ein Werkzeug (30), umfassend
einen Dorn (31) mit Mitteln (37, 38) zum Verbinden mit dem proximalen Abschnitt des ersten Elements des Implantats (2),
eine Hülse (32) mit Mitteln (39) zum Verbinden mit dem zweiten Element des Implantats (2),
einen Verriegelungsdrehknopf (33) zum Feststellen des Werkzeuges (30) in einer definierten Arbeitsposition,
einen Hülsendrehknopf (34) zum Trennen des Werkzeugs (30) vom Implantat (1),
einen Riegel (35) zum Ausklinken des Verriegelungsgriffs (33) und
einen Handgriff (36) zum Drehen des Dorns (31) und/oder der Hülse (32).

10. System gemäß Anspruch 9, **dadurch gekennzeichnet, dass** eine erste Arbeitsposition des Werkzeugs (30) durch Einklinken des Verriegelungsdrehknopfs (33) festgestellt werden kann.

11. System gemäß Anspruch 9 oder 10, **dadurch gekennzeichnet, dass** eine zweite Arbeitsposition des Werkzeugs (30) durch Lösen des Verriegelungsdrehknopfs (33) festgestellt werden kann.

## Revendications

1. Implant (1) pour immobiliser une articulation entre un premier os et un second os, ledit implant comprenant au moins un premier élément (2) et un second élément (3), implant dans lequel le premier élément (2) a un corps cylindrique comprenant :
une section proximale (4) ayant un premier filetage (41),
une section distale (5) comprenant au moins une structure circonférentielle élevée (60), et
une section intermédiaire (7) reliant ladite section proximale (4) et ladite section distale (5), où les sections proximale (4), distale (5) et intermédiaire (7) ont une surface externe (8),
implant dans lequel le second élément (3) a un corps cylindrique comprenant :
une surface externe (15) ayant un deuxième filetage (42),
une surface interne (16) ayant un troisième filetage (43) dont le pas est le même que celui du premier filetage (41), et où le diamètre de la surface interne (16) du second élément (3) est le même que le diamètre de la surface externe (8) du premier élément (2),
implant dans lequel la section intermédiaire (7) du premier élément (2) a au moins un évidement dans la surface externe (8), ledit évidement formant un réservoir (13) approprié pour de la matière osseuse qui est séparée d'un os lors d'un processus d'implantation,
implant dans lequel au moins une structure en forme de canal (12) s'étend depuis l'extrémité frontale (6) du premier élément (2) jusqu'au réservoir (13), et
implant dans lequel la structure circonférentielle (60) est segmentée en au moins un rebord (63), dans une position dans laquelle la structure en forme de canal (12) croise la structure circonférentielle (60), et où le rebord (63) comprend au moins une partie tranchante faisant saillie.

2. Implant (1) selon la revendication 1, **caractérisé en ce que** l'extrémité frontale (6) est formée comme une tête de coupe comprenant au moins une lame de coupe (61) ayant au moins une arête de coupe (62).

3. Implant (1) selon la revendication 2, **caractérisé en ce que** la lame de coupe (61) de l'extrémité frontale est agencée par rapport à la structure en forme de canal (12), de manière telle que la matière osseuse enlevée par la lame de coupe (61) soit poussée directement dans la structure en forme de canal (12).

4. Implant (1) selon l'une quelconque des revendications précédentes, **caractérisé en ce que** le second élément comprend au moins un évidement (19) agencé au niveau de son extrémité faisant face à la section intermédiaire, où l'évidement (19) comprend des parties tranchantes faisant saillie.

5. Implant (1) selon l'une quelconque des revendications précédentes, **caractérisé en ce que** le réservoir (13) formé dans la section intermédiaire (7) comprend des moyens (14) qui sont formés pour diriger, au cours d'un processus d'implantation, un flux de matière partant du réservoir (13) et entrant dans un espace compris entre le premier os et le second os.

6. Implant (1) selon l'une quelconque des revendications précédentes, **caractérisé en ce qu'**au moins une partie des sections proximale (4), distale (5) et / ou intermédiaire (7) comprend une cavité formée par une surface interne (9), où des ouvertures (11) relient la surface externe (8) et la surface interne (9) du premier élément.

7. Implant (1) selon la revendication 6, **caractérisé en ce qu'**un maillage de supports (10) est formé dans la cavité du premier élément (2).

8. Implant (1) selon l'une quelconque des revendications précédentes, **caractérisé en ce que** le premier élément (2) et le second élément (3) ont tous les deux des moyens (17) pour loger un outil qui est formé pour entraîner l'implant.

9. Système comprenant un implant (1) selon l'une quelconque des revendications 1 à 8, et un outil (30) comprenant :
une soie (31) dotée de moyens (37, 38) pour relier à la section proximale du premier élément de l'implant (1),
un manchon (32) doté de moyens (39) pour relier au second élément de l'implant (1),
un bouton de verrouillage (33) pour régler l'outil (30) dans une position de travail définie,
un bouton à douille (34) pour déconnecter l'outil (30), de l'implant (1),
un cliquet (35) pour libérer le bouton de verrouillage (33), et
une poignée (36) pour tourner la soie (31) et / ou le manchon (32).

10. Système selon la revendication 9, **caractérisé en ce qu'**une première position de travail de l'outil (30) peut être réglée en engageant le bouton de verrouillage (33).

11. Système selon la revendication 9 ou 10, **caractérisé en ce qu'**une deuxième position de travail de l'outil (30) peut être réglée en relâchant le bouton de verrouillage (33).
